(19) Europäisches Patentamt
European Patent Office
Office européen des brevets

(11) Veröffentlichungsnummer: **0 123 087**
**B1**

(12)
# EUROPÄISCHE PATENTSCHRIFT

(45) Veröffentlichungstag der Patentschrift: **01.07.87**

(51) Int. Cl.[4]: **C 07 D 277/68,** C 07 D 263/58, C 07 D 235/26

(21) Anmeldenummer: **84102568.7**

(22) Anmeldetag: **09.03.84**

(54) **Verfahren zur Herstellung heterocyclischer Phenylether.**

(30) Priorität: **28.03.83 DE 3311285**

(43) Veröffentlichungstag der Anmeldung: **31.10.84 Patentblatt 84/44**

(45) Bekanntmachung des Hinweises auf die Patenterteilung: **01.07.87 Patentblatt 87/27**

(84) Benannte Vertragsstaaten: **BE CH DE FR GB IT LI NL**

(56) Entgegenhaltungen:
**EP-A-0 000 924**

**C. FERRI: Reaktionen der organischen Chemie, Georg Thieme Verlag, 1978, 395-99**

(73) Patentinhaber: **CASSELLA Aktiengesellschaft, Hanauer Landstrasse 526, D-6000 Frankfurt am Main 61 (DE)**

(72) Erfinder: **Kussmaul, Ulrich, Dr. Dipl.- Chem., Tannenweg 39a, D-6367 Karben 1 (DE)**
Erfinder: **Becherer, Johannes, Dr. Dipl.- Chem., Weidenseestrasse 8, D-6457 Maintal-Bischofsheim (DE)**
Erfinder: **Handte, Reinhard, Dr. Dipl.- Chem., Breckenheimer Strasse 45, D-6238 Hofheim (DE)**
Erfinder: **Müller, Rolf, Dr. Dipl.- Chem., Dornbachweg 3, D-6367 Karben 4 (DE)**

(74) Vertreter: **Urbach, Hans- Georg, Dr., Hanauer Landstrasse 526, D-6000 Frankfurt am Main 61 (DE)**

**EP 0 123 087 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

## Beschreibung

Die vorliegende Erfindung betrifft ein Verfahren zur Herstellung heterocyclischer Phenylether der Formel I

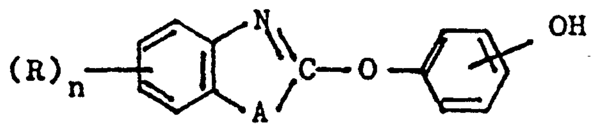

(I)

in der
R gleiche oder verschiedene Reste aus der Gruppe Halogen, $CF_3$, $NO_2$, CN, $(C_1\text{-}C_4)$Alkyl, $(C_1\text{-}C_4)$Alkoxy, $(C_1\text{-}C_4)$Alkylthio,
A O, S oder N-$(C_1\text{-}C_4)$Alkyl und
n 0 bis 3 bedeuten,
wobei eine Verbindung der Formel II

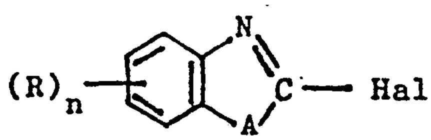

(II)

in der Hal für Halogen, vorzugsweise Cl oder Br steht, oder eine Verbindung der Formel IV

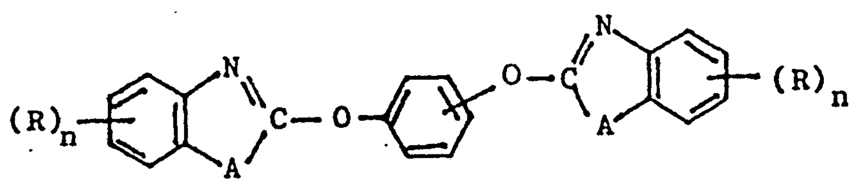

(IV)

mit einer Verbindung der Formel III

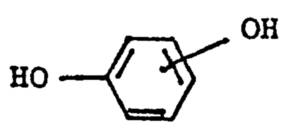

(III)

umgesetzt wird.

Ein solches Verfahren ist bereits aus der europäischen Patentschrift 924 bekannt. Bei diesem bekannten Verfahren wird die Umsetzung bedingungsgemäß in Gegenwart basischer Verbindungen vorgenommen. Die Basen werden dabei in mindestens stöchiometrischen Mengen bzw. einem Überschuß von 10 bis 20 % darüber eingesetzt (vgl. EP-PS 924, Seite 3, Zeilen 34 bis 35). Dieses bekannte Verfahren liefert die heterocyclischen Phenylether der Formel I in guten Ausbeuten, besitzt aber einige Nachteile. So muß nach Beendigung der Reaktion das gebildete Salz abgetrennt werden. Da das abgetrennte Salz noch mit Lösungsmittel behaftet ist, kann es in der abgetrennten Form nicht ohne weiteres entsorgt werden. Ferner muß das nach der Abtrennung des Salzes zurückbleibende Reaktionsgemisch zur Gewinnung der gewünschten Verbindungen I angesäuert werden, was einen zusätzlichen Einsatz von Säure erfordert und die Salzlast des Verfahrens erhöht. Die bei dem bekannten Verfahren verwendeten Lösungsmittel müssen sowohl aus wirtschaftlichen, als auch aus ökologischen Gründen zurückgewonnen werden. Da die Aufarbeitung der Reaktionsansatze in Wasser erfolgt, ist die Rückgewinnung von wassermischbaren Lösungsmittel, z. B. der bevorzugt verwendeten polaren aprotischen Lösungsmittel, in der Regel nur durch eine teure Destillation möglich.

Es wurde nun überraschenderweise gefunden, daß die Nachteile des bekannten Verfahrens vermieden werden können, wenn die Umsetzung in Gegenwart einer Säure und vorzugsweise ohne Lösungsmittel durchgeführt wird.

Nach dem erfindungsgemäßen Verfahren können Verbindungen der Formel II oder Verbindungen der Formel IV oder Gemische von Verbindungen der Formeln II und IV mit Verbindungen der Formel III umgesetzt werden.

Als Säuren werden Lewissäuren oder starke nichtoxidierende Mineralsäuren oder organische Säuren verwendet. Geeignete Mineralsäuren sind z. B. Phosphorsäure oder Halogenwasserstoffe, insbesondere Chlorwasserstoff. Geeignete organische Säuren sind z. B. Trifluoressigsäure oder p-Toluolsulfonsäure. Bezogen auf die Ausgangsverbindungen II oder IV wird die Säure in katalytischer bis äquimolarer Menge eingesetzt. Flüchtige Säuren, wie Halogenwasserstoffe, insbesondere Chlorwasserstoff, werden bevorzugt, weil sie nach beendeter Reaktion durch Temperaturerhöhung und /oder Ausblasen mit einem Inertgas, wie z. B. Stickstoff, leicht aus dem Reaktionsansatz entfernt werden können. Gasförmige Säuren werden während der Reaktion in einem schwachen Strom durch die Reaktionsmischung geleitet.

Werden als Ausgangsprodukte Verbindungen II allein oder im Gemisch mit Verbindungen IV verwendet, dann ist in der Regel der Zusatz einer Säure nicht erforderlich, weil der sich aus der Verbindung II bei der Reaktion bildende Halogenwasserstoff für die Durchführung der Reaktion ausreichend ist. Die Durchführung des erfindungsgemäßen Verfahrens ohne besondere Säurezugabe ist natürlich bevorzugt.

Das erfindungsgemäße Verfahren wird in einem geeigneten Lösungsmittel oder Lösungsmittelgemisch oder vorzugsweise ohne Lösungsmittel durchgeführt. Die benutzten Lösungsmittel müssen gegenüber den Reaktanten inert sein. In Betracht kommen insbesondere wasserunlösliche aromatische Kohlenwasserstoffe und Halogenkohlenwasserstoffe, wie z. B. Toluol, o-, m-, p-Xylol, insbesondere in Form des technischen Xylolgemischs, und Chlorbenzol. Aber auch polare aprotische Lösungsmittel, z. B. Säureamide, wie Dimethylformamid, Dimethylacetamid, Diethylacetamid, N-Methylpyrrolidon und Hexamethylphosphorsäuretriamid, ferner Dimethylsulfoxid oder Nitrile, wie Acetonitril oder Propionitril, können als Lösungsmittel verwendet werden.

Die Reaktionstemperaturen liegen zwischen 50 und 300°C, vorzugsweise bei 100 bis 200°C und, falls ohne Lösungsmittel gearbeitet wird, vorzugsweise oberhalb des Schmelzpunktes der Verbindung III. Bei Reaktionstemperaturen oberhalb des Siedepunkts des verwendeten Lösungsmittels wird die Reaktion in einen geschlossenen Gefäß durchgeführt. Bei dem Einsatz von Ausgangsverbindungen der Formel II allein oder in Gemisch mit Ausgangsverbindungen der Formel IV wird vorzugsweise ohne Zusatz einer Säure gearbeitet. In diesem Fall ist es oft günstig, die Reaktion bei relativ niederer Temperatur durchzuführen, um ein zu rasches Entweichen des bei der Reaktion gebildeten Halogenwasserstoffs aus der Reaktionsmischung zu verhindern. Die während der Reaktion aus dem Reaktionsgemisch entweichenden Halogenwasserstoffe können in bekannter Weise mit Hilfe von Standardapparaten, z. B. in Wasser oder verdünnter Lauge, absorbiert und für andere Einsatzzwecke verwendet werden.

Das Molverhältnis der Reaktanten II : III bzw. IV : III bzw. (II + IV) : III läßt sich in weiten Grenzen variieren. Es ist möglich, mit äquimolaren Mengen zu arbeiten. Günstig ist ein Überschuß der Verbindungen III von mindestens 5 Mol %. Es kann aber auch mit einem doppelt molaren bis 5fach molaren Überschuß an Verbindungen der Formel III gearbeitet werden. Das Molverhältnis der Reaktanten II : III bzw. IV : III bzw. (II + IV) : III beträgt daher normalerweise 1 : (1,05 bis 5), vorzugsweise 1 : (1,05 bis 2). Einen größeren als 5fach molaren Überschuß an der Verbindung III anzuwenden, bringt keinen Vorteil.

Das erfindungsgemäße Verfahren wird im allgemeinen so durchgeführt, daß das Dihydroxybenzol III gegebenenfalls in einem Lösungsmittel und gegebenenfalls mit einer Säure vorgelegt und auf Reaktionstemperatur erwärmt und die Verbindung II oder IV oder ein Gemisch der Verbindungen II und IV zweckmäßigerweise unter Rühren zugegeben wird. Bei Verwendung von Verbindungen II ist, wie bereits erwähnt, die Zugabe einer Säure nicht erforderlich, da nach der Zugabe der Verbindung II kurze Zeit nach Erreichen der Reaktionstemperatur eine lebhafte Entwicklung von Halogenwasserstoff einsetzt. Der aus dem Reaktionsgemisch entweichende Halogenwasserstoff, sei es nun der Überschuß eingeleiteten Halogenwasserstoffs oder während der Reaktion freigesetzter Halogenwasserstoff, wird in bekannter Weise absorbiert. Die Reihenfolge der Zugabe der Reaktanten ist jedoch von untergeordneter Bedeutung; die Reaktanten können auch gleichzeitig zugegeben werden, oder es können die Reaktanten der Formel II bzw. IV vorgelegt werden. Die Vorlage der Verbindungen III ist jedoch bevorzugt. Die Reaktionszeiten sind in allen Fällen kurz und betragen 10 Minuten bis 12 Stunden. Überraschenderweise sind sie jeweils deutlich kürzer als bei den bekannten Verfahren nach EP 924. Der Fortgang der Reaktion kann leicht dünnschichtchromatographisch verfolgt werden. Das erfindungsgemäße Verfahren wird in der Regel unter einem Schutzgas, wie z. B. einem Edelgas, Kohlendioxid oder Stickstoff, durchgeführt. Die Verwendung von Stickstoff als Schutzgas ist bevorzugt. Nach beendeter Reaktion wird bei Verwendung einer flüchtigen Säure diese durch Temperaturerhöhung und/oder Ausblasen mit Schutzgas aus der Reaktionsmischung entfernt. Bei Verwendung eines Lösungsmittels wird der Ansatz abgekühlt und in geeigneter Weise aufgearbeitet. Normalerweise wird der Rückstand abgesaugt und in Wasser eingetragen, wobei durch Zugabe von Lauge (wie z. B. Natron- oder Kalilauge) oder eines Puffers (wie z. B. Dinatriumhydrogenphosphat) dafür gesorgt wird, daß ein pH-Wert von 7 eingehalten wird. Dann wird die Suspension erwärmt bzw. normalerweise aufgekocht und heiß filtriert. Die ohne Verwendung eines Lösungsmittels erhaltenen Ansätze werden in Wasser eingetragen, wobei auch hier durch Zugabe von Lauge oder von Puffern ein pH-Wert von 7 eingehalten wird. Die Suspension wird ebenfalls erwärmt bzw. normalerweise aufgekocht und heiß filtriert.

Durch das Erwärmen in Wasser gehen überschüssige Dihydroxybenzole III in Lösung und können aus dem Filtrat in einfacher und bekannter Weise, z. B. durch Abkühlen und Auskristallisierenlassen, gegebenenfalls nach vorausgegangenem Einengen, wiedergewonnen werden.

Eine weitere Reinigung der erhaltenen Verbindungen I ist für die Weiterverarbeitung in der Regel nicht erforderlich. Gewünschtenfalls kann eine weitere Reinigung durch bekannte Methoden, wie Umkristallisieren, Destillieren oder Umfällen erfolgen.

Die erhaltenen Endprodukte I enthalten in einigen Fällen, je nach dem Molverhältnis der Einsatzprodukte III und II Nebenprodukte der Formel IV. In Fällen, bei denen dieser Gehalt an Verbindungen IV stört, können diese Nebenprodukte leicht aufgrund ihrer Schwerlöslichkeit in Wasser und organischen Lösungsmitteln abgetrennt und in Folgeansätzen als Ausgangsprodukt, eventuell zusammen mit Ausgangsprodukt II, eingesetzt und zu Verbindungen I umgesetzt werden.

Andere Durchführungs- und Aufarbeitungsverfahren sind in den Beispielen beschrieben.

Die mit dem erfindungsgemäßen Verfahren erzielte Produktqualität erreicht in jedem Falle den Stand der Technik oder übertrifft diesen sogar in manchen Fällen.

Das erfindungsgemäße Verfahren wird bevorzugt zur Herstellung solcher Verbindungen der Formel I angewandt, bei denen R gleiche oder verschiedene Reste aus der Gruppe Halogen, insbesondere Fluor, Chlor, Brom, $CF_3$, $NO_2$, Methyl bedeutet, ferner bevorzugt zur Herstellung von Verbindungen I, bei denen $n = 0$, 1 oder 2 bedeutet. Für den Fall, daß in der Formel I A für N-($C_1$-$C_4$)Alkyl steht, bedeutet A vorzugsweise N-$CH_3$.

. Die Verbindungen der Formel I sind wertvolle Vorprodukte für wirksame Biozide, z. B. für Herbizide, insbesondere für solche, wie sie in der US-Patentschrift 4,130,413 vorgeschlagen werden. Gemäß dieser patentschrift erhält man beispielsweise durch Umsetzung mit 2-Halogenpropionsäurederivaten, wie Estern und Amiden, in an sich bekannter Weise aus den Verbindungen der Formel I wertvolle Selektivherbizide. Beispielsweise entsteht aus 4-(6'-Chlorbenzthiazolyl-2'-oxy)-phenol und 2-Brompropionsäureethylester in Gegenwart von Kaliumcarbonat der 2-(4'-(6''-Chlorbenzthiazolyl-2''-oxy)-phenoxy)-propionsäureethylester.

Die Ausgangsverbindungen der Formel II lassen sich nach bekannten Verfahren z. B. aus den entsprechenden 2-Mercapto- bzw. 2-Oxo-Verbindungen durch Halogenierung oder auch aus den 2-Amino-Verbindungen durch Diazotierung und anschließende Sandmeyer-Reaktion herstellen (siehe z. B. C.A. 59,396 j; Am. Chem. J. 21 (1899), 111).

Als Ausgangsverbindungen der Formel II sind verwendbar die 2-Halogenverbindungen entsprechend substituierter Benzthiazole, Benzoxazole und 1-Alkyl-benzimidazole. Beispiele hierfür sind:

2-Chlor-benzoxazol, -1-methyl-benzimidazol; 2-Chlor-6-fluor-benzthiazol, -benzoxazol, -1-methyl-benzimidazol; 2,6-Dichlorbenzthiazol, -benzoxazol, -1-butyl-benzimidazol; 2,5-Dichlor-benzthiazol, -benzoxazol, -1-methyl-benzimidazol; 2-Chlor-5-methyl-benzthiazol, -benzoxazol, -1-methyl-benzimidazol; 2-Chlor-6-methyl-benzthiazol, -benzoxazol, -1-methyl-benzimidazol; 2-Chlor-6-äthyl-benzthiazol, -benzoxazol, -1-methyl-benzimidazol; 2-Chlor-6-nitro-benzthiazol, -benzoxazol, -1-methyl-benzimidazol; 2,5-Dichlor-6-nitro-benzthiazol, -benzoxazol, -1-methyl-benzimidazol; 2-Chlor-5-methoxy-benzthiazol, -benzoxazol, -1-methyl-benzimidazol; 2-Chlor-6-methoxy-benzthiazol, -benzoxazol, -1-methyl-benzimidazol; 2-Chlor-6-methylthio-benzthiazol, -benzoxazol, -1-methyl-benzimidazol; 2,5,6-Trichlor-benzthiazol, -benzoxazol, -1-methyl-benzimidazol; 2-Chlor-5-brom-benzthiazol, -benzoxazol, -1-methyl-benzimidazol; 2-Chlor-6-brom-benzthiazol, -benzoxazol, -1-methyl-benzimidazol; 2-Chlor-5,6-dibrom-benzthiazol, -benzoxazol, -1-methyl-benzimidazol; 2-Chlor-5-trifluormethyl-benzthiazol, -benzoxazol, -1-methyl-benzimidazol; 2-Chlor-6-trifluormethyl-benzthiazol, -benzoxazol, -1-methyl-benzimidazol; 2-Chlor-6-cyano-benzthiazol, -benzoxazol, -1-methyl-benzimidazol, sowie die entsprechenden 2-Bromderivate.

Die Ausgangsverbindungen III, Brenzkatechin, Resorcin und Hydrochinon, sind bekannt. Die Verwendung von Hydrochinon ist bevorzugt.

Die Ausgangsverbindungen der Formel IV entstehen bei der Umsetzung von Verbindungen II mit Verbindungen III als an sich unerwünschte Nebenprodukte, insbesondere dann, wenn bei der Reaktion die Verbindungen II im Überschuß vorhanden sind.

Die Tatsache, daß nach dem erfindungsgemäßen Verfahren unsymmetrische Ether in einfacher und glatter Reaktion aus Hydroxy- und Halogenverbindungen ohne Anwesenheit von Basen hergestellt werden können, muß als äußerst überraschend bezeichnet werden, da man bisher bei der sogenannten Williamson Ethersynthese stets unter Zusatz einer Base oder unter Einsatz von Alkoholat oder Phenolat gearbeitet hat, vgl. C. Ferri: Reaktionen der organischen Synthese (1978), Seite 396.

Die Durchführung des erfindungsgemäßen Verfahrens ohne Lösungsmittel ergibt eine hohe Raumausbeute und erspart andererseits Kosten für die Lösungsmittelregenerierung.

Durch die folgenden Beispiele wird das erfindungsgemäße Verfahren weiter erläutert, ohne es zu beschränken.

**Beispiel 1**

4-(6'-Chlorbenzthiazolyl-2'-oxy)-phenol

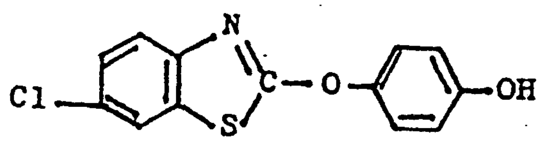

In einem Reaktionsgefäß mit Rührer und Gasableitungsrohr werden unter $N_2$-Schutzgasatmosphäre 220,0 g (2 mol) Hydrochinon aufgeschmolzen. Bei 175°C Innentemperatur läßt man in 25 Minuten 204,1 g (1 mol) geschmolzenes 2,6-Dichlorbenzothiazol unter Rühren zulaufen. Es beginnt eine lebhafte Chlorwasserstoffentwicklung. (Das Abgas wird in üblicher Weise in Wasser zu wäßriger Salzsäure absorbiert.) Nach Ende der Zugabe des 2,6-Dichlorbenzothiazols wird noch 1 h bei 168 bis 170°C nachgerührt. Die dünnschichtchromatographische Kontrolle zeigt dann einen vollständigen Umsatz an.

Zur Aufarbeitung wird der Ansatz sofort noch heiß in fein verteilter Form in 1,5 l gut gerührtes Wasser eingetragen, mit etwas Natronlauge neutralisiert und 10 Minuten bei 100°C gekocht. Es wird heiß abfiltriert und mit wenig heißem Wasser nachgewaschen. Nach Trocknung bei 70°C und einem Druck von ca. 20 mbar erhält man 273,5g (98,5 % der Theorie) 4-(6'-Chlorbenzthiazolyl-2'-oxy)-phenol vom Schmelzpunkt 174 bis 177°C. Umkristallisieren aus Toluol ergibt ein Produkt mit Fp. 178 bis 179°C.

Aus dem erhaltenen Wasserfiltrat fällt nach dem Einengen und Abkühlen überschüssiges Hydrochinon aus. Es wird abfiltriert, nach Trocknung für einen neuen Ansatz verwendet.

**Beispiel 2**

4-(6'-Chlorbenzoxazolyl-2'-oxy)-phenol

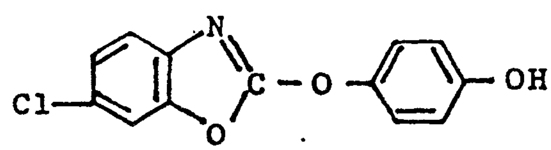

In einem Vierhalskolben mit Rührer, Rückflußkühler, Innenthermometer und Gaseinleitungsrohr werden 200 ml Xylol, 37,6 g (200 mmol)2,6-Dichlorbenzoxazol und 44,0 g (400 mmol)Hydrochinon nach Spülen der Apparatur mit Stickstoff auf 120°C Innentemperatur unter Rühren erhitzt. Das entweichende Chlorwasserstoffgas wird wie im Beispiel 1 absorbiert. Nach 4 h wird zum Austreiben von gelöstem Chlorwasserstoff kurz zum Rückfluß erhitzt und Stickstoff übergeleitet. Der Ansatz wird auf Raumtemperatur abgekühlt und abgesaugt.

Die xylolfeuchte Paste wird mit 100 ml Wasser verrührt, mit ca. 1 bis 1,5 g $Na_2HPO_4$ auf pH 7 gestellt und das Xylol durch Wasserdampfdestillation entfernt. Es wird heiß abgesaugt und mit ca. 150 ml heißem Wasser nachgewaschen. Nach dem Trocknen bei 70°C und ca. 20 mbar erhält man 46,3 g (88 % der Theorie) 4-(6'-Chlorbenzoxazolyl-2'-oxy)-phenol mit einem Fp. von 181 bis 183°C.

Aus dem Filtrat kristallisiert beim Abkühlen überschüssiges Hydrochinon aus.

Wenn bei einer Wiederholung des Beispiels die Reaktionstemperatur auf 135 bis 140°C gesteigert wird, dann verlängert sich die Reaktionszeit auf ca. 8 h. Wenn bei einer Wiederholung des Beispiels die Reaktion durch Kochen am Rückfluß durchgeführt wird, dann ist ohne zusätzliche Zugabe einer Säure keine vernünftige Reaktionsdurchführung mehr möglich, weil der aus dem 2,6-Dichlor-benzoxazol sich bildende Chlorwasserstoff bei der Rückflußtemperatur des Xylols (ca. 144°C) zu rasch aus dem Reaktionsgemisch entweicht.

**Beispiels 3**

4-(6'-Chlorbenzthiazolyl-2'-oxy)-phenol)

60,5 g (0,55 mol) Hydrochinon und 102,0 g (0,5 mol) 2,6-Dichlorbenzothiazol werden analog Beispiel 1 umgesetzt. Das umgesetzte Reaktionsgemisch wird direkt aus Methanol umkristallisiert. Man erhält 124,1 g 4-(6'-Chlorbenzthiazolyl-2'-oxy)-phenol (89,4 % der Theorie) und als Nebenprodukt eine geringe Menge von in Methanol unlöslichem Hydrochinon-1,4-bis-6'-chlorbenzthiazolyl-2'-ether.

**Beispiel 4**

4-(6'-Chlorbenzthiazolyl-2'-oxy)-phenol

8,3 g (75 mmol) Hydrochinon und 11,1 g (25 mmol) Hydrochinon-1,4-bis-6 -chlorbenzthiazolyl-2'-ether und 1 g (5 mmol) 2,6-Dichlorbenzothiazol werden 4 h lang auf 175 bis 180°C erhitzt. Die Aufarbeitung analog Beispiel 1 ergibt 8,0 g (96 % der Theorie) 4-(6'-Chlorbenzthiazolyl-2 -oxy)-phenol.

**Beispiel 5**

4-(6 -Chlorbenzoxazolyl-2'-oxy)-phenol

In einem 2-l-Vierhalskolben mit Rührer, Rückflußkühler, Innenthermometer und Gaseinleitungsrohr werden 1,4 l Xylol, 413 g (1 mol) 1,4-Bis(6'-chlorbenzoxazolyl-2'-oxy)-benzol und 330 g (3 mol) Hydrochinon unter Einleiten eines schwachen HCl-Gasstromes auf 120°C erhitzt und 3 h bei dieser Temperatur gerührt. Dann wird zum Austreiben des HCl-Gases kurz zum Rückfluß erhitzt und etwas Stickstoff durchgeleitet.

Der Ansatz wird auf Zimmertemperatur abgekühlt und abgesaugt. Die xylolfeuchte Paste wird mit 1 l Wasser angerührt, mit ca. 10 bis 15 g $Na_2HPO_4$ auf pH 7 gestellt und dann das Xylol durch Wasserdampfdestillation entfernt. Es wird heiß abgesaugt und mit 1,5 l heißem Wasser nachgewaschen. Nach dem Trocknen verbleiben 475 g 4-(6'-Chlor-benzoxazolyl-2'-oxy)-phenol 95 %ig, Fp. 175 bis 180°C, entsprechend 450 g 100 %ig, das sind 86 % der Theorie. Gehalt an Bisether ca. 4 bis 5 %, Gehalt an Hydrochinon ca. 0,5 %. Aus den Filtrat kristallisiert beim Abkühlen Hydrochinon aus.

**Beispiel 6**

4-(6'-Chlorbenzoxazolyl-2'-oxy)-phenol

In einem 2-l-Vierhalskolben mit Rührer, Rückflußkühler, Innenthermometer und Gaseinleitungsrohr werden 1,4 l Xylol, 228 g (1,2 mol) 2,6-Dichlorbenzoxazol 99 %ig, 165 g (0,4 mol) 1,4-Bis(6'-chlorbenzoxazolyl-2'-oxy)benzol und 396 g (3,6 mol) Hydrochinon nach Spülen der Apparatur mit Stickstoff auf 120 bis 125°C Innentemperatur erhitzt. Im Verlaufe von 3 h entweichen ca. 1,1 mol Chlorwasserstoffgas. Nach Beendigung der Gasentwicklung wird eine weitere Stunde bei 120 bis 125°C gerührt, dann zum Austreiben von gelöstem Chlorwasserstoff kurz zum Rückfluß erhitzt und Stickstoff übergeleitet. Der Ansatz wird auf Raumtemperatur abgekuhlt und abgesaugt.

Die xylolfeuchte Paste wird in 1 l Wasser eingerührt, mit ca. 10 bis 15 g $Na_2HPO_4$ auf pH 7 gestellt und das Xylol durch Wasserdampfdestillation entfernt. Es wird heiß abgesaugt und mit 1,5 l heißem Wasser nachgewaschen. Nach dem Trocknen verbleiben ca. 475 g 4-(6'-Chlorbenzoxazolyl-2'-oxy)-phenol 95 %ig, Fp. 175 bis 180°C, entsprechend ca. 450 g 100 %ig, das sind 86 % der Theorie. Aus dem Filtrat kristallisiert beim Abkühlen Hydrochinon aus.

Gemäß den Beispielen 1 bis 5 erhält man ferner:

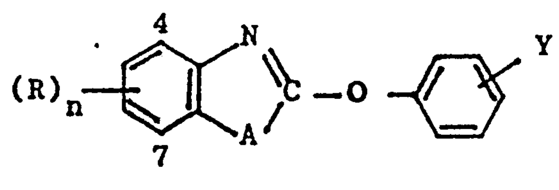

| Beispiel Nr | $(R)_n$ | A | Y |
|---|---|---|---|
| 7 | $5\text{-}CH_3$ | S | 4-OH |
| 8 | $6\text{-}CH_3$ | S | 4-OH |
| 9 | $6\text{-}NO_2$ | S | 3-OH |
| 10 | 5-Br | S | 4-OH |
| 11 | H | $N\text{-}CH_3$ | 4-OH |
| 12 | H | O | 4-OH |
| 13 | H | $N\text{-}CH_3$ | 3-OH |
| 14 | 5-Cl | O | 4-OH |
| 15 | 6-Cl | O | 4-OH |

| | | | |
|---|---|---|---|
| 16 | H | $-N-CH_3$ | 2-OH |
| 17 | 5-Cl 6-$CH_3$ | S | 4-OH |
| 18 | 5,6-di-$CH_3$ | S | 4-OH |
| 19 | 6-$C_2H_5O$ | S | 4-OH |
| 20 | 7-Cl | S | 4-OH |

**Patentansprüche**

1. Verfahren zur Herstellung heterocyclischer Phenylether der Formel I

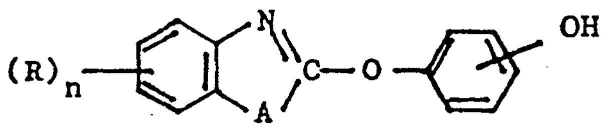

(I)

in der
R gleiche oder verschiedene Reste aus der Gruppe Halogen, $CF_3$, $NO_2$, CN, $(C_1-C_4)$Alkyl, $(C_1-C_4)$Alkoxy, $(C_1-C_4)$Alkylthio,
A O, S oder N-$(C_1-C_4)$Alkyl und
n 0 bis 3 bedeuten,
wobei eine Verbindung der Formel II

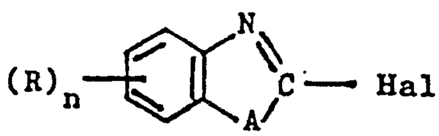

(II)

in der Hal für Halogen, vorzugsweise Cl oder Br steht, oder eine Verbindung der Formel IV

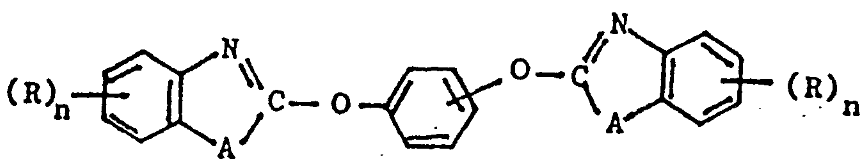

(IV)

mit einer Verbindung der Formel III

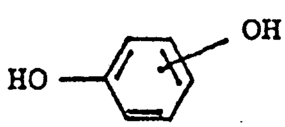

(III)

umgesetzt wird, dadurch gekennzeichnet, daß die Umsetzung in Gegenwart einer Säure erfolgt.

2. Verfahren nach Anspruch 1, dadurch gekennzeichnet, daß die Umsetzung bei Temperaturen von 50 bis 300°C durchgeführt wird.

3. Verfahren nach Anspruch 1 und/oder 2, dadurch gekennzeichnet, daß die Umsetzung bei Temperaturen

von 100 bis 200° C durchgeführt wird.

4. Verfahren nach einem oder mehreren der Ansprüche 1 bis 3, dadurch gekennzeichnet, daß als Säure Chlorwasserstoff verwendet wird.

5. Verfahren nach einem oder mehreren der Ansprüche 1 bis 4, dadurch gekennzeichnet, daß die Umsetzung ohne zusätzliches Lösungsmittel durchgeführt wird.

6. Verfahren nach einem oder mehreren der Ansprüche 1 bis 5, dadurch gekennzeichnet, daß die Umsetzung in der Verbindung III als Lösungsmittel durchgeführt wird.

7. Verfahren nach einem oder mehreren der Ansprüche 1 bis 6, dadurch gekennzeichnet, daß die Umsetzung bei dem Einsatz einer Verbindung der Formel II ohne Zusatz einer Säure durchgeführt wird.

**Claims**

1. Process for the preparation of heterocyclic phenyl ethers of the formula I

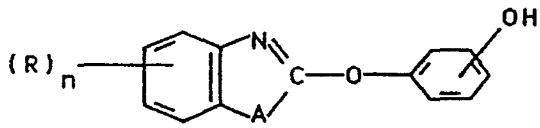

(I)

in which R denotes identical or different radicals from the group comprising halogen, $CF_3$, $NO_2$, CN, $(C_1-C_4)$alkyl, $(C_1-C_4)$alkoxy or $(C_1-C_4)$alkylthio, A denotes O, S or N-$(C_7-C_4)$alkyl, and n denotes 0 to 3, which entails reacting a compound of the formula II

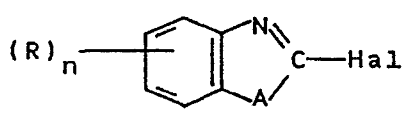

(II)

in which Hal represents halogen, preferably Cl or Br, or a compound of the formula IV

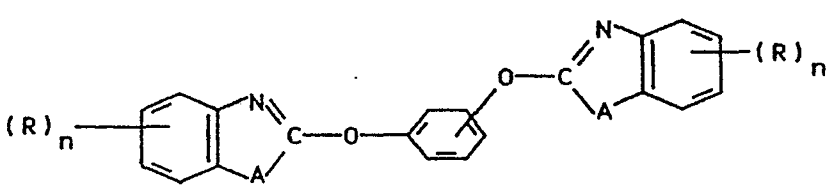

(IV)

with a compound of the formula III

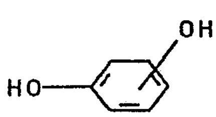

(III)

characterised in that the reaction is carried out in the presence of an acid.

2. Process according to Claim 1, characterised in that the reaction is carried out at temperatures from 50 to 300° C.

3. Process according to Claim 1 and/or 2, characterised in that the reaction is carried out at temperatures from 100 to 200° C.

4. Process according to one or more of Claims 1 to 3, characterised in that hydrogen chloride is used as the acid.

5. Process according to one or more of Claims 1 to 4, characterised in that the reaction is carried out without additional solvent.

6. Process according to one or more of Claims 1 to 5, characterised in that the reaction is carried out in the compound III as the solvent.

7. Process according to one or more of Claims 1 to 6, characterised in that, when a compound of the formula II is employed, the reaction is carried out without the addition of an acid.

**Revendications**

1. Procédé pour préparer des éthers phényliques hétérocyliques de formule I

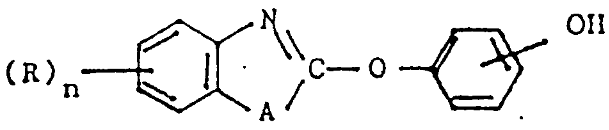

(I)

dans laquelle

R représente des radicaux identiques ou différents du groupe comprenant les halogènes, $CF_3$, $NO_2$, CN, les radicaux alkyle en $C_1$-$C_4$, alcoxy en $C_1$-$C_4$, alkylthio en $C_1$-$C_4$,

A représente O, S ou un radical N-(alkyle en $C_1$-$C_4$), et

n vaut 0 à 3,

dans lequel on fait réagir un composé de formule II

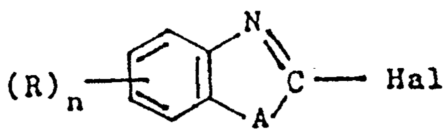

(II)

dans laquelle Hal désigne un halogène, de préférence Cl ou Br, ou un composé de formule IV

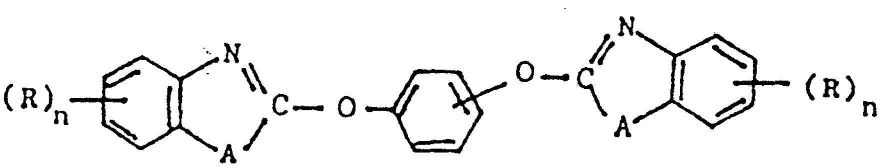

(IV)

sur un composé de formule III

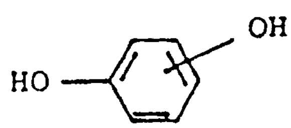

(III)

caractérisé en ce que la réaction se déroule en présence d'un acide.

2. Procédé selon la revendication 1, caractérisé en ce que la réaction se déroule à des températures de 50 à 300° C.

3. Procédé selon la revendication 1 et/ou 2, caractérisé en ce que la réaction se déroule à des températures de 100 à 200°C.

4. Procédé selon l'une ou plusieurs des revendications 1 à 3, caractérisé en ce qu'on utilise comme acide de l'acide chlorhydrique.

5. Procédé selon l'une ou plusieurs des revendications 1 à 4, caractérisé en ce que la réaction se déroule sans addition d'un solvant.

6. Procédé selon l'une ou plusieurs des revendications 1 à 5, caractérisé en ce que la réaction se déroule dans le composé III servant de solvant.

7. Procédé selon l'une ou plusieurs des revendications 1 à 6, caractérisé en ce qu'on procède à la réaction en utilisant un composé de formule II sans addition d'un acide.